# EUROPEAN PATENT APPLICATION

(11) **EP 0 940 138 A1**
(43) Date of publication of application: **08.09.1999**
(21) Application number: 97929525.0
(22) Date of filing: 04.07.1997
(51) Int. Cl.: A61K 9/02, A61K 47/14, C07C 69/24, C07C 67/08, C07C 67/54

(54) **LUBRICATION ASSISTANT AND PROCESS FOR THE PREPARATION OF ETHYL STEARATE USED THEREIN**

(30) Priority: 04.07.1996 JP 17447696
(71) Applicant: Seiken Chemical Co., Ltd., Ichikawa-shi, Chiba 272 (JP); Yoshimizu, Kimiko, Tokyo 152 (JP)
(72) Inventor: YOSHIMIZU, Kimiko, Tokyo 152 (JP)
(74) Representative: Votier, Sidney David
(86) International application number: JP9702323
(87) International publication number: WO9801113

(57) **Abstract**

A supplementary lubricating body which lubricates the entire surface of a mucous membrane when inserted into a body cavity such as the vagina. Furthermore it protects the mucous membrane from objects inserted from the outside. It is pleasant to use and is form mainly from ethyl stearic acid.

## Description

### Field of the Invention

The present invention relates to a supplementary lubrication body used for insertion into a body cavity such as the anus or the vagina.

### Background of the Invention

Up until the present time, the moisture on the mucous membrane of body cavities such as the vagina decreases with age. In particular, with old age, there is little moisture on the mucous membrane and when articles for use during menstruation or other articles are inserted and removed, pain is experienced due to the scraping on the mucous membrane of the vaginal wall. Furthermore during constipation or the like, stools do not easily slide from the bowel and bowel movements are difficult. In such cases, various kinds of oil or jelly are spread on the body openings such as the anus or vagina to increase the lubrication. Furthermore in persons with reduced defecation functions such as the sick, in order to excrete hard stools, there is a danger of scarring on the mucous membrane of the bowel wall due to assisting the passage of the stool with the finger.

Moreover since such bodily apertures are in lower parts of the body that are out of sight and are lubricated with market-sold gel type oils or jelly, it is difficult and inconvenient to lubricate only the bodily aperture without soiling clothes. Since marketed oils and jellies have a high viscosity, they are very sticky and are difficult to use due to their smell. Also since they are usually packaged in a tube form or the like, they tend to stand out and it is difficult to carry them without drawing attention.

The present invention is proposed to solve the above problems and has as its purpose the provision of a suppository form lubricant which may be inserted into body cavities as necessary. This kind of product for example is disclosed for example in JP-A-61-176522. This product is comprised of a polyoxyethylene glycol component which is in liquid form below 75° C and has a molecular weight of 200-20,000, with a non-ionic surface active agent and a lubricant. It is in a lubricating suppository form, maintains its solidity from 10-60° C when exposed to air and dissolves into a uniform liquid containing water when in the body.

However this lubricating suppository uses water soluble compounds in all compounds and all compounds used have a viscosity higher than, for example, 300 centistoke. As a result, since such compounds differ greatly from the mucus which is excreted by the mucous membrane of the vaginal wall of a human being (which could be termed bodily fluids which have a viscosity of 20-60 centistoke), the sticky sensation creates an unpleasant impression when used. Furthermore, since such conventional lubricating suppositories do not melt due to the body temperature but dissolve due to moisture absorption of the water components from the mucus membrane, the problem has arisen that if the bodily fluids from the mucous membrane of the vagina wall are insufficient, time will be needed for their dissolution.

Thus the present invention has the purpose of providing a lubricating supplementary body which takes advantage of the fact that individual differences and age differences exert no great difference on the temperature of body cavities of adult human beings, which lubricates the entire mucous membrane of the body cavity on being inserted into a body cavity such as the vagina, which protects the mucous membrane from objects inserted from the outside and which is pleasant to use.

### Disclosure of the Invention

In order to achieve the above object, embodiment 1 of the present invention comprises a lubricating supplementary body which is characterized by having a main body formed from ethyl stearic acid.

In embodiment 2, the lubricating supplementary body of the first embodiment is characterized by the fusion point of ethyl stearic acid being in the range of 30-35° C.

In embodiment 3, the lubricating supplementary body of the first embodiment is characterized by the purity of the ethyl stearic acid being in the range of 90-99% by weight.

In embodiment 4, the lubricating supplementary body of the first embodiment is characterized by the viscosity of the ethyl stearic acid of being 35 centistoke at 36° C.

In embodiment 5, the lubricating supplementary body of the first embodiment is characterized by the shape of the lubricating supplementary body being spherical, bar shaped, elliptical, hemispherical, conical, fusiform, round, oval, suppository shaped or capsule shaped.

In embodiment 6, the lubricating supplementary body of the fifth embodiment is characterized by all shapes lacking corners.

In embodiment 7, the lubricating supplementary body of the fifth embodiment is characterized by a lubricating supplementary body which has a weight of about 1-5 g.

In embodiment 8, the supplementary lubricating body with respect to any one of embodiments 1-6 is characterized by ethyl stearic acid containing a medicament.

In embodiment 9, the supplementary lubricating body with respect to any one of embodiments 1-8 is characterized by ethyl stearic acid containing a germicidal agent.

In embodiment 10, the supplementary lubricating body with respect to any one of embodiments 1-9 is characterized by said ethyl stearic acid being covered by a film.

In embodiment 11, the supplementary lubricating body with respect to embodiment 10 is characterized by said film being a heat sealable film and said ethyl stearic acid being contained in said film by heat fusion and said ethyl stearic acid thus contained being removable by breaking the heat sealed section.

In embodiment 12, the supplementary lubricating body with respect to embodiment 10 is characterized by said film being a soil capsule and said ethyl stearic acid being contained by said soil capsule the periphery of which is heat sealed and said soil capsule being composed on a basis of 50-95% by weight of gelatin and 5-40 % by weight of glycerin, and 1-15% by weight of water.

In embodiment 13, the supplementary lubricating body with respect to embodiment 12 is characterized by said soft capsule being cross linked.

In embodiment 14, the supplementary lubricating body with respect to embodiment 13 is characterized by said cross linking of said soft capsule being carried out by any one of acetaldehyde, propylene oxide, ethylene oxide, epicholorohydrin, glyoxal and glutaraldehyde.

In embodiment 15, the supplementary lubricating body with respect to embodiment 1 is characterized by said ethyl stearic acid being formed by reacting ethyl-alcohol with stearic acid using concentrated sulfuric acid as a catalyst, then the resultant powder being released in water, and being purified by rinsing and distilling under reduced pressure.

In embodiment 16, the supplementary lubricating body with respect to any one of embodiment 1-15 is characterized by being used by insertion into a body cavity having a mucous membrane.

Embodiment 17 is a method of manufacturing ethyl stearic acid in which ethyl alcohol and stearic acid are reacted using concentrated sulfuric acid as a catalyst, then is released into water and rinsed, and is purified by distilling under reduced pressure.

In embodiment 18, the method of manufacturing ethyl stearic acid with respect to embodiment 17 is characterized by the additional step of steam distillation after the step of distilling under pressure.

### Brief Explanation of the Drawings

Figure 1 is a perspective view of a supplementary lubricating body according to an embodiment of the present invention.
Figure 2 is a perspective view of a split mold for manufacturing a supplementary lubricating body according to an embodiment of the present invention.
Figure 3 is a cross section of an example of the application of packaging to the supplementary lubrication body.
Figure 4 is a perspective view of the packaging material used in Figure 3.
Figure 5 is another example of use of the present invention and shows a soft capsule containing solid ester stearic acid. Figure 5(a) is a plan view and Figure 5(b) is a front view.
Figure 6 is another example of use of the present invention and shows a soft capsule containing solid ester stearic acid. Figure 6(a) is a plan view and Figure 6(b) is a front view.

In the figures, reference numeral 1 denotes a supplementary lubricating body, 2 is a bullet shaped projection, 3 is a split mold, 4 is packaging member, 5 is a soft capsule, 5a is a surface, 6 is a clip.

### Preferred Embodiments of the Invention

The present invention will be explained on the basis of the figures, however it is not of course limited by those figures.

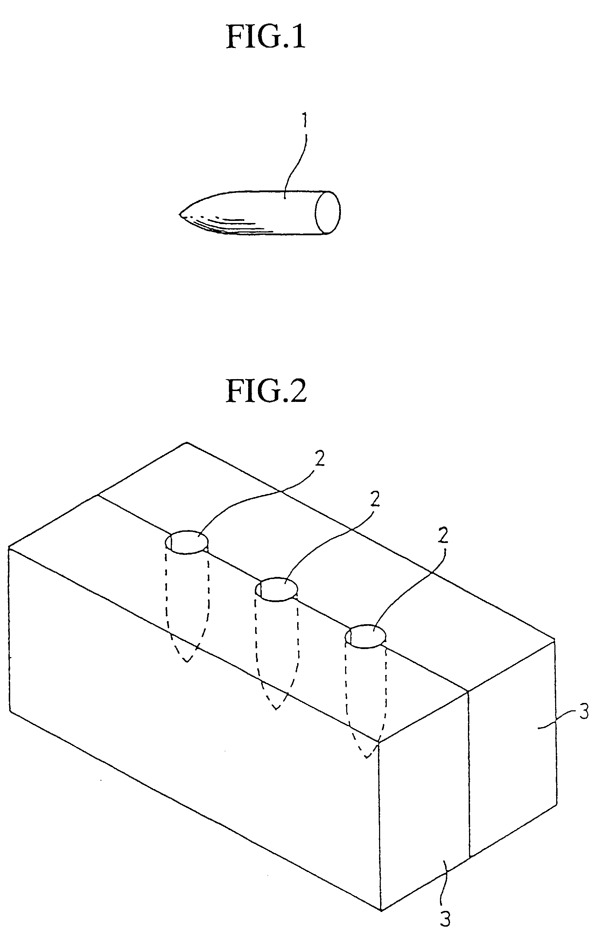

Figures 1 and 2 show a first aspect of an embodiment of the present invention. In the figures, 1 is a lubricating supplementary body according to the present invention formed in suppository form by a method of manufacture outlined below. So that there is little resistance when inserted into the vagina and so as not to injure the mucous membrane, for example, it is formed in a round shape without edges.

The lubricating supplementary body 1 is formed by relatively pure ethyl stearic acid. Furthermore ethyl stearic acid is a white crystalline solid which is soluble in ether or alcohol, insoluble in water and non-odorous (since there is no problem in practice using substances with some odor, substances having some odor are treated here as if having no odor). Ethyl stearic acid when manufactured to 100% purity has a fusion point of 33.7-35° C.

It is entered on the Japanese Cosmetics Ingredient Dictionary at I-79, is recognized as an ingredient for manufacture of cosmetics and at the present time is used and sold in creams and hairdressing preparations.

The fusion point of ethyl stearic acid increases with increasing purity and decreases with decreasing purity. The ethyl stearic acid used preferentially in the present invention has a fusion point of 30-35° C when it has a purity of 90-99.9 % by weight.

The high purity ethyl stearic acid used in the present invention is normally obtained by esterification of marketed high purity stearic acid (for example 98%) using ethyl alcohol and sulfuric acid and is made as discussed below.

In other words, 40g of anhydrous ethyl alcohol is placed into a 1 liter round flask, while this is agitated, 45g of concentrated sulfuric acid with a relative density of 1.84 is added. 300g of high purity stearic acid (for example ACIDCHEM PALMAC 98-18) is dissolved in this mixture and the mixture is heated in a water bath for 1 hour while being passed through a reflux condenser. Then ice-containing water is poured and the precipitated powder form ethyl stearic acid is separated by filtration and the sulfuric acid is washed off. It is heated to 40-50° C and the water is removed. The oil is distilled in a 10mm Hg vacuum and a fraction is extracted at 195-205° C. The ethyl stearic acid obtained by the above process has a fusion point of 33.7° C and a viscosity of 35 centistoke at 36° C.

As above, the distillation at a low pressure is necessary to remove the smell generated when there is a slight residue of low fatty acid esters in the ethyl stearic acid of the present invention. In products in which quality permits a slight smell, the above step is not always necessary. An effective method for removing low fatty acid esters is steam distillation.

Many kinds of ethyl stearic acid sold on the market contain many impurities and have a low solidifying point. The ethyl stearic acid used in the supplementary lubrication body of the present invention is of high purity and has a sufficiently high temperature to allow liquefaction at body temperature. This type of relatively pure ethyl stearic acid solidifies if cooled to a temperature lower than normal and can easily be shaped into a desired form. Furthermore since there is no variation in the fusion point or mechanical properties of the ethyl stearic acid if it is once melted and then quickly re-cooled, even if it is melted and only several usable amounts are removed, it can be quickly cooled, solidified and used.

When inserted into a body cavity such as the anus or vagina, it is desirable that it dissolve in 20-30 minutes. Naturally periods longer or shorter than this are also acceptable. As a result, a fusion point of about 30-35° C is desirable and a weight of 1-5g or particularly 3g is desirable. In other words, a single dosage of ethyl stearic acid of 1-5g is desirable and particularly about 3g is desirable. Although ethyl stearic acid with a fusion point above 30 ° C differs as regards purity depending on the kinds of impurities present, a purity of more than 93% by weight as a rule is necessary. Ethyl stearic acid with a purity of in the range of 90-99% by weight and preferentially in the range of 98-99% by weight is used. Ethyl stearic acid with a purity of above 99% by weight has a high price and is not practical on a cost/performance basis. On this point, ethyl stearic acid with a fusion point of more than 33° C and less than 35° C is preferable.

In this aspect of the embodiment, a split mold 3, 3 formed from a material such as plastic or soft gelatin and having a bullet shaped projection as shown in Figure 2 is used. Liquid ethyl stearic acid is poured into this split mold 3,3. This liquid ethyl stearic acid is placed in a glass or stainless container in powder form (or lump form) and has a fusion point of 33-35° C. For example it is melted by a hot bath at 60° C and in this molten state is allowed to flow into the split mold 3,3. It is then placed into a refrigerator or the like and cooled to 5-10° C to solidify the molten ethyl stearic acid. After this, the split mold 3,3 is removed and a bullet shaped supplementary lubrication body is formed (the fusion method). The weight of a single supplementary lubrication body is about 3g as a standard. However this may differ depending on the constitution of user or the race of the user.

The shape of the supplementary lubrication body is not limited to the shape as discussed in this embodiment. It could be formed into an optional shape for example round, elliptical, hemispherical, cylindrical, fusiform, oval, capsule or bar shaped. It can be in tablet form so as to be used as a vaginal tablet.

When in capsule form, manufacture is facilitated by the addition of a small amount of a polymer compound such as methyl-cellulose to the ethyl stearic acid in order to strengthen the coating of the ethyl stearic acid. An optional medicament or the like can be packed into this capsule shape.

The supplementary lubrication body 1 is normally formed from 98-99% pure by weight ethyl stearic acid on a cost/performance basis. When placed stored at normal temperatures or held lightly in the palm of the hand, it does not easily melt since body heat is not transmitted to the entire surface of the solid ethyl stearic acid and thus it maintains its original shape. Furthermore after insertion into a body cavity such as the vagina, body heat is transmitted to the entire surface of the solid ethyl stearic acid by the mucous membrane of the vagina. Since the temperature of the supplementary lubrication body is maintained at a temperature higher than its fusion point, the solid ethyl stearic acid melts and becomes a liquid body with low viscosity. It spreads throughout the body cavity such as the vagina and lubricates the entirety of the mucous membrane and inner walls. The periphery of the body aperture is also lubricated by the outflow of the ethyl stearic acid. For example, the body melts in 20 minutes in a person with a high body temperature and in 30 minutes in a person with a normal body temperature.

When the high purity ethyl stearic acid of the present invention is in a molten state in the body cavity, impurities in the body cavity can lower the fusion point of the ethyl stearic acid and even if the body comes into contact with the outside air with a temperature lower than body temperature, it is difficult to harden. In this way, cream may be applied to the skin in the periphery of the body cavity aperture to lubricate the skin.

Normally the basic constituent of suppositories is cacao butter or tri-glyceride fatty acids. Such substances when melted have high viscosity in the region of 200-1600 centistoke and thus create a sticky sensation when used. Furthermore cacao butter is a compound of various fats and fatty oils. Since its fusion point varies over a wide range, it is difficult to use due to the fact that when held in the palm of the hand for example, fats and fatty oils forming a part of the surface melt or change shape. As a result, it is normal to place it in a normal household refrigerator for safekeeping.

The ethyl stearic acid used in the present invention is insoluble in water, and is a large molecule having a molecular weight of 312.5. It is not dispersed through the mucous membranes in the body cavities such as the vagina and is maintained in the body cavities. Therefore the supplementary lubrication body used in accordance with the present invention can be stored for a relatively long time and maintains the mucous membranes on the walls of body cavities in a lubricated state. This fluid body is pleasant to use as it has a slippery texture (which is similar to the fluids secreted from body cavities such as the vagina) and is not sticky. Furthermore this substance does not irritate the skin and has no influence on the human body. Thus it can be used generally as above, and is recognized as having long term safety.

Thus when an object inserted into a body cavity such as a vagina and there is a danger of an irritation or pain due to friction between the inserted body and the mucous membrane forming the body cavity wall. If the supplementary lubrication body 1 is inserted about 5-10 min before the insertion, the supplementary lubrication body 1 is slowly melted by body heat and lubricates the entire surface of the mucous membrane in the body cavity. Thus the insertion of the object or its withdrawal is smoothly performed and the mucous membrane is not damaged. Furthermore when fluid flows outside the body, the ethyl stearic acid prevents the inserted object from re-congealing as a result of its solidifying point being lowered by changes due to impurities already existing in the body cavity such as a vagina. As a result, any ethyl stearic acid which flows outside the body can be easily wiped with paper such as a soft tissue.

In order to melt the supplementary lubrication body with body heat, the fusion point of ethyl stearic acid is lower than body temperature, for example it must be below 36° C. If the fusion point is too low, since melting will occur too quickly and ease of use will be impaired, it is preferable that the fusion point be in the range between 30-35° C.

There are many compounds with a fusion point in the range 30-35° C. Furthermore by mixing in other compounds with a high fusion point it is possible to regulate the fusion point of the compound in question to the range 30-35° C by the phenomenon of depression of fusion point.

For example, apart from ethyl stearic acid, examples of compounds with a fusion point in the range 30-35° C are laurate heptacosyl lauric acid, benzyl-naphthalene, dibenzyl-ketone, eicosane, methyl-naphthalene, tetra-decylalcohol. These simple substances are molten at body temperature, they may be used in the present invention. However on consideration of their safety, skin irritation characteristics, skin absorbency, smell, hygroscopicity, viscosity after melting, and lubricative properties, they can not be used without some modification. For example heptacosyl lauric acid has a smell. Benzyl naphthlene, dibenzyl-ketone, methyl-naphthalene are aromatic compounds which may be carcinogenic. Eicosane has a high viscosity.

Cocao butter, sawarii fat, glycero-gelatine, macrogol, triglycerides, fatty acids such as Witepsol which are used in other suppositories are known. These however have high viscosity, and in all cases a condition such as applicability, viscosity after melting, lubrication or user friendliness is not satisfied.

In order to increase the value and stability of the formulation of the supplementary lubrication body 1, various kinds of medicaments such as hormones, antibiotics, chemotherapeutics, Chinese medicines or the like may be added, or oxidation inhibitors (anti-oxidants), bactericides, anti-mould agents, stabilizers or anti-bacterial/mould agents may be added to the degree that they do not impair the object of the present invention. Preferably dibutyl-hydroxytoluene or vitamin E can be used as anti-oxidants. P-hyroxy-ethyl-benzoate, p-hydroxy-methyl-benzoate, p-hydroxy-butyl-benzoate, p-hydroxy-isopropyl-benzoate or di-iodomethyl-p-tolylsulfon can be used as anti-bacterial/mould agents. A kind of yellow ginger used in Chinese medicine called "toukishigyakukagosu" is suggested as a heat insulating material.

Furthermore pigments, aromas or bulking agents may be added. The ethyl stearic acid used in the present invention can be used as a suppository basis to be mixed with cocao butter or the like and this substance can be used without the addition of surfactants or other excipients. Furthermore it is possible to adapt conventional suppositories by mixing with ethyl stearic acid, to a degree that does not detract from the purpose of the present invention, such substances as surfactants, suppository bases, excipients or the like which are used in suppository form or are known suppositories. Such additives or blends for example may be taken from the FDA, the Japanese Pharmacopoeia, the Japanese Pharmacopoeia of Standards for Medicines for External Use, Standards for Drug Additives, Standards for Quasidrug Ingredients but are not so limited. In addition muscarine may be added.

Since Chinese medicines which are not soluble in ethyl stearic acid usually do not depress the fusion point (solidification point) and therefore may be used by mixing large amounts. Since medically active substances such as vitamin E or γ-oryzanol depress the fusion point of ethyl stearic acid, it is necessary to consider the addition of large amounts carefully. However it is generally possible to add 0.01-0.1% of these substances without trouble.

If types of ovarian hormones (for example estrogen, estradiol, estriol) are added, the supplementary lubricating body 1 will have an efficacy with respect to osteoporosis or the like. It is also possible to add additives effective against all kinds of bacteria or viruses.

As stated above, it is possible to depress the fusion temperature of ethyl stearic acid by mixing in other substances. By exploiting this characteristic, it is possible to positively depress the fusion point by mixing in other substances and use the supplementary lubricating body 1 in low temperature parts of the body or animals with a low body temperature.

The shape of the supplementary lubricating body 1 is maintained when its surface, for example a water soluble film, is in a cold state. However it is possible to enhance the stability of the shape with a capsule shaped covering in the form of a film which releases the ethyl stearic acid from within on melting in the body. In this case, it is possible to freely choose the additives or complexes added to the ethyl stearic acid packed inside. For example even if the fusion point of ethyl stearic acid is lowered by more than 33° C below the fusion point, use is still possible.

Furthermore it is possible to pack such kinds of supplementary lubricating bodies in a film or foil such as paraffin paper, aluminum foil or the like after it is formed. When packed in the previously mentioned water soluble films, or the above films or foils, even if the contents melt on being kept at a temperature of 40 ° C, the packaging will maintain the shape of the supplementary lubricating bodies 1 which may be placed in that state in a cool place, hardened and put in a usable state.

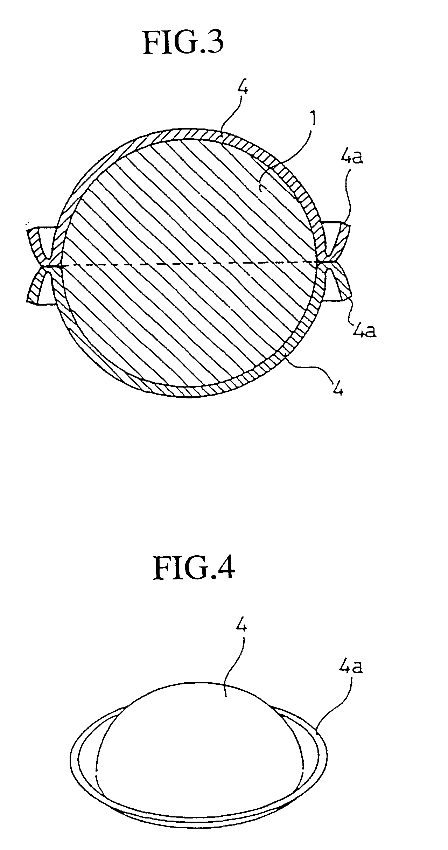

As an application of the present invention, the supplementary lubricating body 1 may be formed in a round shape as shown in Figure 3. In this case, the round supplementary lubricating body 1 as shown in Figure 4 may be covered by a covering means from the upper and lower directions with a pair of films 4,4 which are heat sealable and are formed in a hemispherical shape. The respective halves of the pair of films 4,4 form a flange 4a covering the entire peripheral aperture of the hemispherical film 4. The affixed base of the flange 4a, 4a is heat sealed with a metal mold along the entire periphery after the round solidified supplementary lubricating body 1 is packaged from the upper and lower directions. When formed in this way, it is easy to remove the contents which comprise the solidified supplementary lubricating body 1 during use by pinching the tip of the flange 4a, 4a with a fingers and simply removing the solidified supplementary lubricating body 1 by peeling of the covering. Of course it is needless to say that the flange 4a, 4a need not be provided around the entire periphery.

The appearance of the film 4 used as a packaging may be enhanced by the addition of colorants, fluorescent agents or the like and the product may have an increased value. In this way, if a fluorescent agent is added to the packaging material, it is possible to see the packaging material at night even if the surroundings are dark. The ethyl stearic acid formed in an optional shape such as a suppository shape, a round shape or a bar shape may be covered with a covering membrane (hereafter a soft capsule) which has a uniform thickness and is formed by a basic water soluble component of 50-90% by weight of gelatin, and 5-40% by weight glycerin and 1-15% by weight of water. The surface may be cross linked with such compounds as acetaldehyde, propylene oxide, ethylene oxide, epichlorohydrin, glyoxal and glutaraldehyde.

When cross bonding is performed, a crack may be easily created in the surface of the soft capsule which is a flexible membrane (including substances with very low flexibility) by applying a torsion force from the outside holding it with the fingers of both hands. That is to say the contact surface of the solid ethyl stearic acid and the soil capsule is merely touching and is easily broken. Thus when a torsion force is applied, the force does not affect the surface of the ethyl stearic acid. The covering membrane breaks, the ethyl stearic acid is released and use is easily performed. Furthermore since this object dissolves in water, after use, disposal is conveniently carried out.

Since the heat expansion co-efficient of the soft capsule and the ethyl stearic acid is approximately the same, after the ethyl stearic acid is placed seamlessly in the soil capsule, even if it is placed under high temperatures and the ethyl stearic acid melts and expands, the soft capsule expands in the same way and thus the ethyl stearic acid continues to be contained seamlessly in the container. Thus even if it is re-cooled in a refrigerator, it will solidify to its original shape without any formation of edges. As a result, since there are no edges in re-solidified goods, no injury to the mucous membrane results.

In addition if a material which does not change shape even under relatively high temperatures is used as the container material, if the body is covered so that air can not substantively enter the container, even if the body melts and re-solidifies it will return to its original shape.

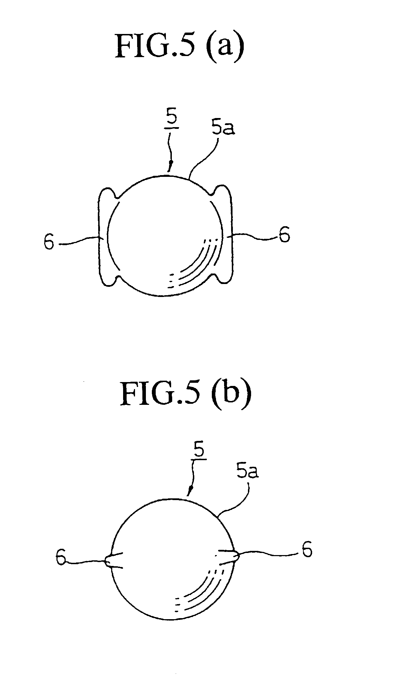

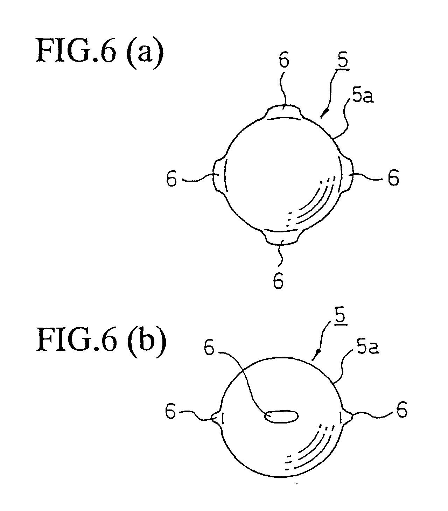

The procedure below should be carried out in order to make the soft capsule easy to grasp in the fingers. For example, as shown in Figure 5, gripping sections 6 are provided on the left and right hand sides of the surface 5a of the round soft capsule. Furthermore as shown in Figure 6, it is possible to provide gripping sections 6 at four positions on the periphery of the soft capsule. These gripping sections 6 have a relatively thick wall on the base section as opposed to the tip. In this case, by bending the left/right pair of gripping sections 6 of the cross linked soft capsule in the opposite direction, the surface 5a breaks and it is easy to remove the solid ethyl stearic acid which forms the contents.

The soil capsule 5 is made by for example pouring a fixed amount of molten ethyl stearic acid into a container which comprises half opened pair of metallic molds in which a cylindrical shaped film with a bottom, which forms the material of the soft film has been placed. After pouring, it is shaped and sealed and manufactured by completely closing the pair of half open metallic molds.

Shrinkable materials such as rubber membranes, vinylchloride or chlorine treated rubber may be considered for this kind of covering membrane and the body can be packaged with them. In this case, the shrinkage material may be shrunk during or after packaging by elasticity or heat contraction and thus made to closely adhere to the contents.

The supplementary lubricating body 1 of the present invention was explained above as being inserted into the vagina. However it may be applied to the case of an aged person, who suffers from constipation due to the abdominal muscle weakening due to sickness, extracting a stool with a finger. In this latter case, the insertion into the anus about 10 min. prior to extraction will give lubrication to the intestinal wall. Even if a finger is inserted into the anus, since the surface of the mucous membrane is slippery, the finger can be inserted deeply into the anus without any friction with the mucous membrane or any sensation of pain. Furthermore the base material of the supplementary lubricating body may be made insertable. In this way, the supplementary lubricating body of the present invention can use an instrument or a finger to insert into body cavities such as the anus or vagina and it is possible to increase the lubrication in the body cavity.

Below the aspects of the embodiments are shown. However in the aspects of the embodiments below the % unless indicated otherwise mean % by weight.

### Embodiment 1

The prescribed amounts below constitute the supplementary lubricating body 1 by the fusion method as shown in Figure 1.

The ethyl stearic acid used is manufactured by the method disclosed in the present document and a refined type is used. All additives No.1 - No.8 of the supplementary lubricating body 1 approximately are decomposed equivalently. Their fusion point is in the range 30-35° C and do not melt when held in the palm of the hand. On the other hand, when they melt, they exhibit a pleasant sleek texture. In contrast, No. 10 has a low fusion point and thus a part of the surface melts and becomes sticky when in the palm of the hand.

### Prescribed Amounts for a Supplementary Lubricating Body

| (No. 1) | |
|---|---|
| Ethyl stearic acid | 99.9% |
| Dibutyl-hydroxtoluene | 0.05% (stabilizer, antioxidant) |
| p-chloro-m-xylenol (chlorocresol) | 0.05%(anti-mould/bacterial) |

| (No.2) | |
|---|---|
| Ethyl stearic acid | 99.48% |
| Vitamin E (tocopherol) | 0.3% (stabilizer, antioxidant) |
| p-chloro-m-xylenol | 0.02%(anti-mould/bacterial) |
| Fragrance | 0.2% |

| (No.3) | |
|---|---|
| Ethyl stearic acid | 98.8% |
| Di-iodomethyl-p-tolylsulfon | 1.0%(anti-mould/bacterial) |
| Dibutyl-hydroxtoluene | 0.1%(stabilizer, antioxidant) |
| p-hydrox-ethylbenzoate | 0.1%(anti-bacterial) |

| (No.4) | |
|---|---|
| Ethyl stearic acid | 99.27% |
| Cefabuperazone | 0.5%(antibiotic) |
| γ-oryzanol | 0.1%(antioxidant) |
| p-hydrox-methylbenzoate | 0.1%(anti-bacterial) |
| p-hydrox-butylbenzoate | 0.03% (anti-bacterial) |

| (No. 5) | |
|---|---|
| Ethyl stearic acid | 99.65% |
| Ofloxacin | 0.2% (chemotherapeutic) |
| Dibutyl-hydroxtoluene | 0.05%(stabilizer, antioxidant) |
| p-hydrox-isopropylbenzoate | 0.1%(anti-bacterial) |

| (No.6) | |
|---|---|
| Ethyl stearic acid | 99.75% |
| Toukishigyakukagosu | 0.1%(Chinese medicine) |
| Dibutyl-hydroxtoluene | 0.1%(stabilizer, antioxidant) |
| p-chloro-m-xylenol | 0.05%(anti-mould/bacterial) |

| (No.7) | |
|---|---|
| Ethyl stearic acid | 99.815% |
| Estriol | 0.02%(Ovarian hormone) |
| Dibutyl-hydroxtoluene | 0.02%(stabilizer, antioxidant) |
| p-hydrox-methylbenzoate | 0.05%(anti-bacterial) |

| (No.9) | |
|---|---|
| Cacao Butter | 99.9% |
| Dibutyl-hydroxtoluene | 0.05%(stabilizer, antioxidant) |
| p-chloro-m-xylenol | 0.05%(anti-mould/bacterial) |

Experiments No.1 and No.9 were conducted in water at a temperature of 34° C. No. 1 was stable and showed no surface change. However No. 9 began to melt on the surface after about a minute and after 10 minutes had melted to a degree that it did not hold its shape when held in the palm of the hand.

When these two were further placed into a water bath at 37° C, No. 1 completely melted after 10 minutes but No. 9 was not completely melted after 10 minutes.

### Industrial Applicability

As explained above, according to the present invention, a supplementary lubricating body is provided which is easy to use, safe, and maintains its shape at normal temperatures. When inserted into body cavities, it melts and becomes a liquid body having appropriate viscosity which spreads throughout the entire body cavity. Thus the mucous membrane of the body cavity is lubricated. As it is not soluble in water, it is not easily absorbed in the body cavity and thus has high stability. Therefore if the supplementary lubricating body according to the present invention is used, the mucous membrane of body cavities can maintain lubrication for relatively long periods of time. Furthermore the liquid body has a smooth texture and thus is pleasant to use. At present as this product is used as a cosmetic and is recognized to be safe, it has no harmful side effects on the human body.

## Claims

1. A supplementary lubricating body which is mainly formed from ethyl stearic acid.

2. A supplementary lubricating body as defined by claim 1 wherein a melting point of said ethyl stearic acid is in the range 30-35° C.

3. A supplementary lubricating body as defined by claim 1 wherein the purity of said ethyl stearic acid is in the range 90-99% by weight.

4. A supplementary lubricating body as defined by claim 1 wherein the viscosity of said ethyl stearic acid at 36° C is approximately 35 centistoke.

5. A supplementary lubricating body as defined by any one of claims 1 to 4 wherein the shape of said supplementary lubricating body is spherical, bar-shaped, elliptical, hemi-spherical, conical, fusiform, round, oval, suppository or capsule shaped.

6. A supplementary lubricating body as defined by claim 5 wherein each shape of said supplementary lubricating body does not have edges.

7. A supplementary lubricating body as defined by claim 5 wherein said supplementary lubricating body has a weight of approximately 1-5g.

8. A supplementary lubricating body as defined by any one of claims 1 to 6 wherein said ethyl stearic acid contains a medicament.

9. A supplementary lubricating body as defined by any one of claims 1 to 8 wherein said ethyl stearic acid contains an antibiotic.

10. A supplementary lubricating body as defined by any one of claims 1 to 9 wherein said ethyl stearic acid is covered by a film.

11. A supplementary lubricating body as defined by claim 10 wherein said film is a heat sealable film and said ethyl stearic acid is contained within said heat sealed film and said ethyl stearic acid can be removed from within by peeling the film.

12. A supplementary lubricating body as defined by claim 10 wherein said film is a soft capsule and said ethyl stearic acid is contained by the periphery of said soft capsule being heat sealed and said soft capsule is formed by 50-95% by weight of gelatin, 5-40% by weight of glycerin, and 1-15% by weight of water.

13. A supplementary lubricating body as defined by claim 12 wherein said soft capsule is cross linked.

14. A supplementary lubricating body as defined by claim 13 wherein said soft capsule cross linking process is performed by any one of acetaldehyde, propylene oxide, ethylene oxide, epicholorohydrin, glyoxal and glutaraldehyde.

15. A supplementary lubricating body as defined by claim 1 wherein said ethyl stearic acid is manufactured by reacting ethyl-alcohol with stearic acid using concentrated sulfuric acid as a catalyst, then the resultant powder is released in water, and rinsed and then refined by distilling under reduced pressure.

16. A supplementary lubricating body as defined by any one of claims 1 to 15 wherein said supplementary lubricating body is inserted into a body cavity having a mucous membrane.

17. A method of manufacturing ethyl stearic acid by reacting ethyl-alcohol with stearic acid using concentrated sulfuric acid as a catalyst, then the resultant powder is released in water, and rinsed and then refined by distilling under reduced pressure.

18. A method of manufacturing ethyl stearic acid as defined by claim 17 wherein after said distilling under reduced pressure, the process includes the additional step of steam distillation.
